(19) Europäisches Patentamt
European Patent Office
Office européen des brevets

(11) **EP 3 892 191 A1**

(12) **EUROPEAN PATENT APPLICATION**

(43) Date of publication:
**13.10.2021 Bulletin 2021/41**

(51) Int Cl.:
*A61B 5/053* (2021.01)   *G01R 33/56* (2006.01)
*G01R 33/563* (2006.01)   *G01R 33/44* (2006.01)

(21) Application number: **20168971.8**

(22) Date of filing: **09.04.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **KATSCHER, Ulrich**
  **5656 AE Eindhoven (NL)**
• **KEUPP, Jochen**
  **5656 AE Eindhoven (NL)**

(74) Representative: **Philips Intellectual Property & Standards**
**High Tech Campus 5**
**5656 AE Eindhoven (NL)**

(54) **MEASUREMENT OF INTRA-CELLULAR CONDUCTIVITY USING MAGNETIC RESONANCE IMAGING**

(57) Disclosed herein is a method of medical imaging. The method comprises: receiving (200) a first electrical properties tomography conductivity map (122) for a first radio frequency value, receiving (202) a second electrical properties tomography conductivity map (124), calculating (204) a low frequency conductivity map (126) using the first electrical properties tomography conductivity map and the second electrical properties tomography conductivity map, calculating (208) an intra-cellular volume map (130) and an extra-cellular volume map (131) by performing an optimization of a two-term exponential model to the multi-b diffusion weighted magnetic resonance imaging signal spectra map, calculating (210) an intra-cellular volume fraction map (132) by performing a voxel wise division of the intra-cellular volume map by the voxel wise sum of the extra-cellular volume map plus the intra-cellular volume map, and calculating (212) an intra-cellular conductivity map (134) by performing a voxel wise division of an intermediate mapping by the intra-cellular volume fraction map.

Fig. 6

**Description**

FIELD OF THE INVENTION

The invention relates to magnetic resonance imaging, in particular to electrical properties tomography.

BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Various quantities or properties of the subject can be measured spatially using MRI. For example, in a technique knows as Electrical Properties Tomography (EPT) distortions of the measured B1 field can be used to reconstruct electrical properties of a subject, such as spatially dependent conductivity maps.

The journal article Sajib et. al., "Electrodeless conductivity tensor imaging (CTI) using MRI: basic theory and animal experiments," Biomedical Engineering Letters, 25 Apr 2018, 8(3):273-282, DOI: 10.1007/s13534-018-0066-3 discloses an electrodeless method of conductivity tensor imaging (CTI) utilizes B1 mapping to recover a high-frequency isotropic conductivity image which is influenced by contents in both extracellular and intracellular spaces. Multi-b diffusion weighted imaging is then utilized to extract the effects of the extracellular space and incorporate its directional structural property.

SUMMARY OF THE INVENTION

The invention provides for a magnetic resonance imaging system, a computer program product and a method in the independent claims.

Embodiments may provide for a more accurate means of measuring the spatially dependent intra-cellular conductivity. This may be accomplished by making multiple electrical properties tomography measurements at different radio-frequency values. The radio-frequency (the Lamour frequency) at which spins (hydrogen protons) precess at is determined by the B0 magnetic field strength. Measurements at multiple radio-frequency values can therefore be achieved, for example, by making multiple EPT measurements in different B0 fields. The use of electrical properties tomography conductivity maps from two different radio-frequency values enables the calculation of an approximate low frequency conductivity map. This in turn enables, as disclosed herein, a separate calculation of the spatially dependent intracellular conductivity and spatially dependent extracellular conductivity.

The invention provides for a medical system, a method, and a computer program in the independent claims. Embodiments are given in the dependent claims.

In one aspect the invention provides for a medical system that comprises a memory storing machine-executable instructions. The medical system further comprises a computational system that is configured for controlling the medical system.

Execution of the machine-executable instructions causes the computational system to receive a first electrical properties tomography conductivity map descriptive of a region of interest of a subject. The first electrical properties tomography conductivity map is for a first radio-frequency value. Conductivity may be measured as a function of frequency. The first electrical properties tomography conductivity map is made for a first frequency that is in the radio-frequency spectrum.

Execution of the machine-executable instructions further causes the computational system to receive a second electrical properties tomography conductivity map descriptive of the region of interest of a subject. The second electrical properties tomography conductivity map is for a second radio-frequency value or measured at a second radio-frequency value. The first electrical properties tomography conductivity map is registered to the second electrical properties tomography conductivity map. The registration may for example be a map which spatially relates voxels of one conductivity map to the other.

Execution of the machine-executable instructions further causes the computational system to calculate a low-frequency conductivity map using the first electrical properties tomography conductivity map and the second electrical properties tomography conductivity map. A knowledge of the conductivity at two radio-frequency values enables a reasonable estimate of the low-frequency conductivity map.

Execution of the machine-executable instructions further causes the computational system to receive a multi-b diffusion weighted magnetic resonance imaging signal spectra map. The multi-b diffusion weighted magnetic resonance imaging signal spectra map is registered to the first electrical properties tomography conductivity map or the second electrical properties tomography conductivity map. Execution of the machine-executable instructions further causes the computational system to calculate an intra-cellular volume map and an extra-cellular volume map by performing an optimization of a two-term exponential model to the multi-b diffusion weighted magnetic resonance imaging signal spectra map.

Execution of the machine-executable instructions further causes the computational system to calculate an intra-cellular volume fraction map by performing a voxel-wise division of the intra-cellular volume map by the voxel-wise sum of the extra-cellular volume map plus the intra-cellular volume map. The term voxel-wise as used herein means that these various mathematical functions are performed between two voxels. For example, determining which two voxels are used may be done by using the spatial registration between the first electrical properties tomography conductivity map and the second electrical properties tomography conductivity map.

Execution of the machine-executable instructions further causes the computational system calculate an intra-cellular conductivity map by performing a voxel-wise division of an intermediate mapping by the intra-cellular volume fraction map. The intermediate mapping is the voxel-wise difference of a selected radio-frequency conductivity map and the low-frequency conductivity map. The selected radio-frequency conductivity map is one of the first electrical properties tomography conductivity map and the second electrical properties tomography conductivity map.

The term low-frequency map could for example be for a frequency that is between approximately 100 Hz and 300 kHz.

This embodiment may be beneficial because it enables the calculation of an intra-cellular conductivity map. This is the conductivity within the cells. This may be beneficial because it may provide insight into the state or pathology of the cells.

In another embodiment execution of the machine-executable instructions further causes the computational system to calculate an extra-cellular volume fraction map by performing a voxel-wise division of the intra-cellular volume map by the voxel-wise sum of the extra-cellular volume map plus the intra-cellular volume map. Execution of the machine-executable instructions further causes the computational system to calculate an extra-cellular conductivity map by performing a voxel-wise division of the lower-frequency conductivity map by the extra-cellular volume fraction map. Having the extra-cellular conductivity map may also be beneficial because it may provide a means of classifying regions of the subject as having a particular pathology. For example, the conductivity may go up as the sodium content goes up. This may be useful for example in assisting a physician in detecting tumors or other tissue types.

In another embodiment execution of the machine-executable instructions further causes the computational system to render the intra-cellular conductivity map. The intra-cellular conductivity map may for example be rendered adjacent to another magnetic resonance image or it may also be overlaid on a different magnetic resonance image. This may aid the physician or other user to interpret the magnetic resonance images or data.

In another embodiment the intra-cellular conductivity map is rendered as an overlay over a magnetic resonance image. The intra-cellular conductivity map is registered to the magnetic resonance image.

In another embodiment the calculation of the low-frequency conductivity map using the first electrical properties tomography conductivity map and the second electrical properties tomography conductivity map is performed as an extrapolation according to a Cole-Cole model. The use of a Cole-Cole model may be useful because in the radio-frequency range using a double-exponential presentation the conductivity is essentially a linear value plus a constant. The measurement of the conductivity maps at two frequencies then enables an accurate estimate of the low-frequency conductivity map.

In another embodiment the optimization of the two-term exponential model to the multi-b diffusion weighted magnetic resonance imaging signal spectra map also provides an intra-cellular diffusion constant map and an extra-cellular constant map. The combination of the conductivity and the diffusion provide further information about the pathology of the subj ect.

Execution of the machine-executable instructions further causes the computational system to perform any one of the following: calculate an intra-cellular ion concentration map using the intra-cellular conductivity map and the intra-cellular diffusion constant map; calculate an extra-cellular ion concentration map using the extra-cellular conductivity map and the extra-cellular diffusion constant map; and combinations thereof.

In another embodiment the intra-cellular ion concentration map and/or the extra-cellular ion concentration map are temperature corrected using any one of the following: a constant body temperature of the subject and a magnetic resonance thermometry temperature map of the region of interest. In many cases the body temperature of the subject will be relatively constant and stable. In this case the temperature of the subject may be assumed or a measurement of the temperature of the subject may be measured and this may be used.

In another embodiment execution of the machine-executable instructions further causes the computational system to receive first electrical properties tomography k-space data descriptive of the region of interest for a first proton resonance frequency matching the first radio-frequency value. Execution of the machine-executable instructions further causes the computational system to receive second electrical properties tomography k-space data descriptive of the region of interest for a second proton resonance frequency matching the second radio-frequency value. Execution of the machine-executable instructions further causes the computational system to receive multi-b diffusion weighted k-space data descriptive of the region of interest with either the first proton resonance frequency or the second proton resonance frequency.

Execution of the machine-executable instructions further causes the computational system to reconstruct the first electrical properties tomography conductivity map from the first electrical properties tomography k-space data. Execution of the machine-executable instructions further causes the computational system to reconstruct the second electrical properties tomography conductivity map from the second electrical properties tomography k-space data. Execution of the machine-executable instructions further causes the computational system to reconstruct the multi-b diffusion weighted magnetic resonance imaging signal spectra map from the multi-b diffusion weighted k-space data.

In another embodiment execution of the machine-executable instructions further causes the computational system to register the first electrical properties tomography conductivity map, the second electrical properties tomography conductivity map, and the multi-b diffusion weighted magnetic resonance imaging signal spectra map to each other.

In another embodiment the medical system further comprises a magnetic resonance imaging system configured for acquiring k-space data for at least two distinct B0 magnetic field values. The at least two distinct magnetic field values have a proton resonance frequency matching the first radio-frequency value and the second radio-frequency value within the imaging zone. The memory further contains first pulse sequence commands configured for controlling the magnetic resonance imaging system to acquire the first electrical properties tomography k-space data. The memory further contains second pulse sequence commands configured for controlling the magnetic resonance imaging system to acquire the second electrical properties tomography k-space data.

The memory further contains third pulse sequence commands configured for controlling the magnetic resonance imaging system to acquire the multi-b diffusion weighted k-space data. Execution of the machine-executable instructions further causes the computational system to control the magnetic resonance imaging system to acquire the first electrical properties tomography k-space data by controlling the magnetic resonance imaging system with the first pulse sequence commands. Execution of the machine-executable instructions further causes the computational system to control the magnetic resonance imaging system to acquire the second electrical properties tomography k-space data by controlling the magnetic resonance imaging system with the second pulse sequence commands.

Execution of the machine-executable instructions further causes the computational system to control the magnetic resonance imaging system to acquire the multi-b diffusion weighted k-space data by controlling the magnetic resonance imaging system with the third pulse sequence commands.

In another embodiment the magnetic resonance imaging system comprises a main magnet for each of the at least two distinct B0 magnetic field values. In this embodiment there are essentially two magnetic resonance imaging systems.

In another embodiment the magnetic resonance imaging system comprises a variable magnetic field main magnet configured for adjusting the B0 magnetic field to each of the at least two distinct B0 magnetic field values. For example, the current in the main magnet may be adjusted to adjust the B0 magnetic field for each of the measurements of k-space data. This may have the advantage that the subject can have the data measured while the subject is in the same location. This may make it easier to register the various images and maps to each other.

In another aspect the invention provides for a method of medical imaging. The method comprises receiving a first electrical properties tomography conductivity map descriptive of a region of interest of a subject. The first electrical properties tomography conductivity map is for a first radio-frequency value. The method further comprises receiving a second electrical properties tomography conductivity map descriptive of the region of interest of a subject. The second electrical properties tomography conductivity map is for a second radio-frequency value. The first electrical properties tomography conductivity map is registered to the second electrical properties tomography conductivity map. The method further comprises calculating a low-frequency conductivity map using the first electrical properties tomography conductivity map and the second electrical properties tomography conductivity map.

The method further comprises receiving a multi-b diffusion weighted magnetic resonance imaging signal spectra map. The multi-b diffusion weighted magnetic resonance imaging signal spectra map is registered to the first electrical properties tomography conductivity map or the second electrical properties tomography conductivity map. The method further comprises calculating an intra-cellular volume map and an extra-cellular volume map. This is done by performing an optimization of a two-term exponential model to the multi-b diffusion weighted magnetic resonance imaging signal spectra map.

The method further comprises calculating an intra-cellular volume fraction map by performing a voxel-wise division of the intra-cellular volume map by the voxel-wise sum of the extra-cellular volume map plus the intra-cellular volume map. The method further comprises calculating an intra-cellular conductivity map by performing a voxel-wise division of an intermediate mapping by the intra-cellular volume fraction map. The intermediate mapping is the voxel-wise difference of a selected radio-frequency conductivity map and the low-frequency conductivity map. The selected radio-frequency conductivity map is one of the first electrical properties tomography conductivity map and the second electrical properties tomography conductivity map.

In another aspect the invention provides for a computer program comprising machine-executable instructions for execution by a computational system controlling a medical system. In some examples the computer program may be a computer program product that may for example be stored on a non-transitory storage medium.

Execution of the machine-executable instructions causes the computational system to receive a first electrical properties tomography conductivity map descriptive of a region of interest of a subject. The first electrical properties tomography conductivity map is for a first radio-frequency value. The method further comprises receiving a second electrical properties tomography conductivity map descriptive of the region of interest of a subject. The second electrical properties tomography conductivity map is for a second radio-frequency value. The first electrical properties tomography conductivity map is registered to the second electrical properties tomography conductivity map.

Execution of the machine-executable instructions further causes the computational system to calculate a low-frequency conductivity map using the first electrical properties tomography conductivity map and the second electrical properties tomography conductivity map. Execution of the machine-executable instructions further causes the computational system to receive a multi-b diffusion weighted magnetic resonance imaging signal spectra map. The multi-b diffusion weighted magnetic resonance imaging signal spectra map is registered to the first electrical properties tomography conductivity map or the second electrical properties tomography conductivity map.

Execution of the machine-executable instructions further causes the computational system to calculate an intra-cellular volume map and an extra-cellular volume map. This is done by performing an optimization of a two-term exponential model to the multi-b diffusion weighted magnetic resonance imaging signal spectra map. Execution of the machine-executable instructions further causes the computational system to calculate an intra-cellular volume fraction map by performing a voxel-wise division of the intra-cellular volume map by the voxel-wise sum of the extra-cellular volume map plus the intra-cellular volume map. Execution of the machine-executable instructions further causes the computational system to calculate an intra-cellular conductivity map by performing a voxel-wise division of an intermediate mapping by the intra-cellular volume fraction map. The intermediate mapping is the voxel-wise difference of a selected radio-frequency conductivity map and the low-frequency conductivity map. The selected radio-frequency conductivity map is one of the first electrical properties tomography conductivity map and the second electrical properties tomography conductivity map.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image or MR image is defined herein as being the reconstructed two- or three-dimensional visualization of anatomic data contained within the magnetic resonance imaging data. This visualization can be performed using a computer.

BRIEF DESCRIPTION OF THE DRAWINGS

In the following preferred embodiments of the invention will be described, by way of example only, and with reference to the drawings in which:

Fig. 1 illustrates an example of a medical system;
Fig. 2 shows a flow chart which illustrates a method of operating the medical system of Fig. 1;
Fig. 3 illustrates a further example of a medical system;
Fig. 4 illustrates a further example of a medical system;
Fig. 5 illustrates an example of a method; and
Fig. 6 illustrates a further example of a method.

DETAILED DESCRIPTION OF THE EMBODIMENTS

Like numbered elements in these figures are either equivalent elements or perform the same function. Elements which have been discussed previously will not necessarily be discussed in later figures if the function is equivalent.

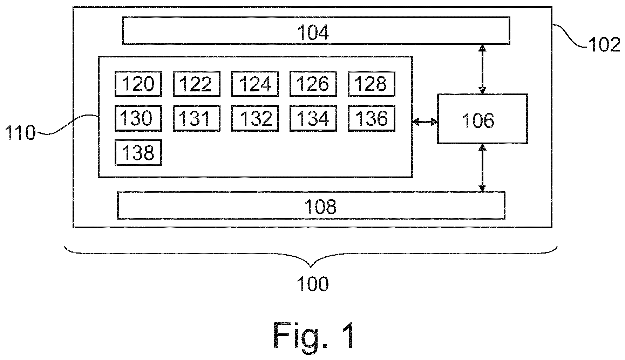

Fig. 1 illustrates an example of a medical system 100. The medical system is shown as comprising a computer 102 which is used to house a computational system 106. The computer 102 may represent one or more computers that are networked or connected together. The computational system 106 may represent one or more processors or devices which are capable of performing computational tasks. This may also include such things as microprocessors, controllers, and programmable field gate arrays.

The computational system 106 may be distributed in multiple locations. The computational system 106 is shown as being connected to a hardware interface 104. The hardware interface may enable the computational system 106 to control other components of the medical system 100 as well as communicate with other computational systems and/or computers. The computational system 106 is shown as being connected to an optional user interface 108. The computational system 106 is further shown as being connected to a memory 110. The memory 110 represents one or more memory devices that are accessible to the computational system 106. The memory 110 may for example be a non-transitory storage medium in some examples.

The memory 110 is shown as containing machine-executable instructions 120. The machine-executable instructions 120 may contain instructions which enable the computational system 106 to control the operation and function as well as other components of the medical system 100. The machine-executable instructions 120 may also enable the computational system 106 to perform data and image processing tasks. The memory is further shown as containing a first electrical properties tomography conductivity map 122 and a second electrical properties tomography conductivity map 124. The memory 110 is further shown as containing a low-frequency conductivity map 126 that was calculated using the first electrical properties tomography conductivity map 122 and the second electrical properties tomography conductivity map 124.

The memory 110 is further shown as containing a multi-b diffusion weighted magnetic resonance imaging signal spectra map 128. The memory 110 is further shown as containing an intra-cellular volume map 130 and an extra-cellular volume map 131. These two maps 130, 131 were calculated by performing an optimization of a two-term exponential model to the multi-b diffusion weighted magnetic resonance imaging signal spectra map 128. The memory is further shown as containing an intra-cellular volume fraction map 132. The intra-cellular volume fraction map was calculated by performing a voxel-wise division of the intra-cellular volume map 130 by the voxel-wise sum of the extra-cellular volume map 131 plus the intra-cellular volume map 130. The memory is further shown as containing an intra-cellular conductivity map 134 that was calculated by performing a voxel-wise division of an intermediate mapping by the intra-cellular volume fraction map 132. The intermediate mapping is the voxel-wise difference of a selected radio-frequency conductivity map and the low-frequency conductivity map 126. The selected radio-frequency conductivity map is one of the first electrical properties tomography conductivity map 122 and second electrical properties tomography conductivity map 124.

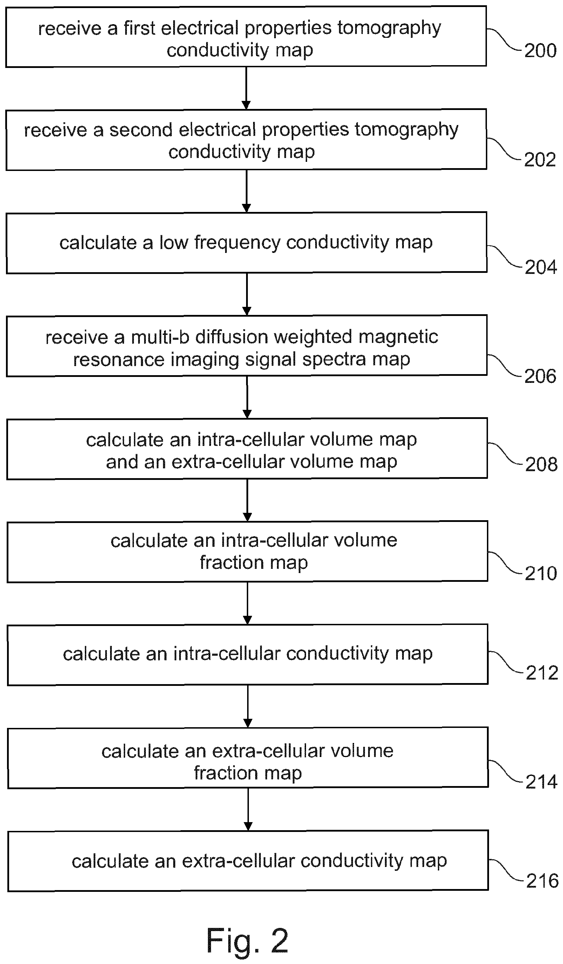

Fig. 2 shows a flowchart which illustrates a method of operating the medical system of Fig. 1. First in step 200 the first electrical properties tomography conductivity map 122 is received. Next, in step 202, the second electrical properties tomography conductivity map 124 is received. Both are descriptive of a region of interest of a subject. The first electrical properties tomography conductivity map 122 is a conductivity map at a first radio-frequency value. The second electrical properties tomography conductivity map 124 is a conductivity map at a second radio-frequency value. There is also a registration between the first electrical properties tomography conductivity map 122 and the second electrical properties tomography conductivity map 124.

Next, in step 204, a low-frequency conductivity map 126 is calculated from the first electrical properties tomography conductivity map 122 and the second electrical properties tomography conductivity map 124. Then, in step 206, the multi-b diffusion weighted magnetic resonance imaging signal spectra map 128 is received. This spectra map 128 is registered to either the first electrical properties tomography conductivity map 122 or the second electrical properties tomography conductivity map 124. Then, in step 208, the intra-cellular volume map and the extra-cellular volume map 131 are calculated. This is done by performing an optimization of a two-term exponential model to the multi-b diffusion weighted magnetic resonance imaging signal spectra map 128.

In step 210, the intra-cellular volume fraction map 132 is calculated by performing a voxel-wise division of the intra-cellular volume map by the voxel-wise sum of the extra-cellular volume map plus the intra-cellular volume map. Then, in step 212, the intra-cellular conductivity map 134 is calculated by performing a voxel-wise division of the intermediate mapping by the intra-cellular volume fraction map 132. The intermediate mapping is the voxel-wise difference of a selected radio-frequency conductivity map and the low-frequency conductivity map 126. The selected radio-frequency conductivity map is one of the first electrical properties tomography conductivity map 122 and the second electrical properties tomography conductivity map 124.

Steps 214 and 216 are optional. In step 214 an extra-cellular volume fraction map 136 is calculated by performing a voxel-wise division of the intra-cellular volume map 130 by the voxel-wise sum of the extra-cellular volume map 131 plus the intra-cellular volume map 130. Then finally, in step 216, the optional extra-cellular conductivity map 138 is calculated by performing a voxel-wise division of the low-frequency conductivity map 126 by the extra-cellular volume fraction map 136.

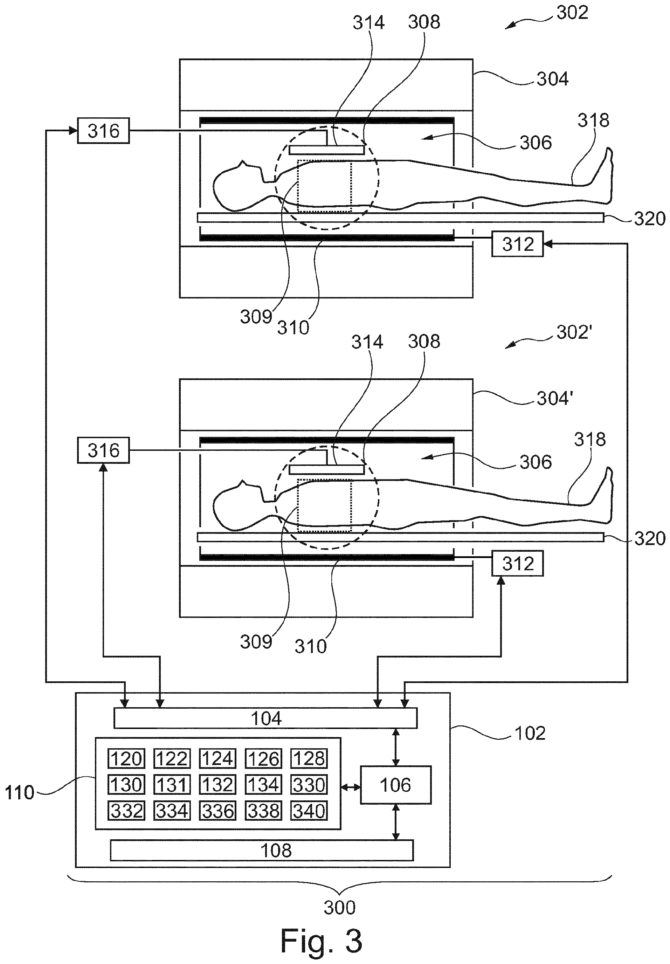

Fig. 3 illustrates a further example of a medical system 300. The medical system 300 is similar to the medical system 100 in Fig 1, except the medical system 300 comprises two separate magnetic resonance imaging systems 302, 302'. The first magnetic resonance imaging system 302 comprises a first magnet 304 and the second magnetic resonance imaging system 302' comprises a second magnet 304'. The first magnet 304 is configured for generating a first B0 magnetic field in the imaging zone 308 and the second magnet 304' is configured for generating a B0 magnetic field with a different field strength when within its imaging zone 308. These two distinct B0 magnetic field values correspond to the first radio-frequency value and the second radio-frequency value for the electrical properties tomography conductivity maps 122 and 124. The common features of the magnetic resonance imaging systems 302 and 302' are described together.

The magnet 304, 304' may be a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304, 304' there is an imaging zone 308 where the magnetic field is strong and uniform enough to perform magnetic resonance imaging. The value of the B0 field within the imaging zone 308 differs between the two magnets 304, 304'. A region of interest 309 is shown within the imaging zone 308.

The same subject 318 is shown as in the bore 306 of both magnets 304, 304'. The subject 318 is shown as being supported by a subject support 320 such that at least a portion of the subject 318 is within the imaging zone 308 and the region of interest 309. The magnetic resonance imaging systems 302, 302' may be controlled so that the region of interest 309 between the two is as similar as possible. In some instances, the data acquired in two magnetic resonance imaging systems 302, 302' are registered to each other. This may be done after a reconstruction using standard image processing techniques.

Within the bore 306 of the magnet there is also a set of magnetic field gradient coils 310 which is used for acquisition of preliminary magnetic resonance data to spatially encode magnetic spins within the imaging zone 308 of the magnet 304, 304'. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receiver. The radio-frequency coil 314 may also have multiple receive/transmit elements and the radio frequency transceiver 316 may have multiple receive/transmit channels. For example if a parallel imaging technique such as SENSE is performed, the radio-frequency could 314 will have multiple coil elements.

The transceiver 316 and the gradient controller 312 of both magnetic resonance imaging systems 302, 302' are shown as being connected to the hardware interface 106 of a computer system 102.

The memory 110 is shown as containing the first pulse sequence commands 330. The first pulse sequence commands 330 are configured for controlling the first magnetic resonance imaging system 302 to acquire the first electrical properties tomography k-space data 336 which is also shown as stored in the memory 110. The second pulse sequence commands 332 are configured for controlling the second magnetic resonance imaging system 302' to acquire second electrical properties tomography k-space data 338.

The first electrical properties tomography k-space data 336 may be reconstructed into the first electrical properties tomography conductivity map 122. The second electrical properties tomography k-space data 338 may be reconstructed into the second electrical properties tomography conductivity map 124. The memory 334 is also shown as comprising third pulse sequence commands 334. The third pulse sequence commands 334 may be used for controlling either the first magnetic resonance imaging system 302 or the second magnetic resonance imaging system 302' to acquire multi-b diffusion weighted k-space data 340. The multi-b diffusion weighted k-space data 340 may be used to reconstruct the multi-b diffusion weighted magnetic resonance imaging signal spectra map 128.

In Fig. 3 the computer system 102 is depicted as a single unit. However, the computer system 102 may in fact be multiple computer systems. For example, there may be a workstation or computer controlling each of the magnetic resonance imaging systems 302, 302' individually. There may also be an additional computer system 102 that is used for image reconstruction later.

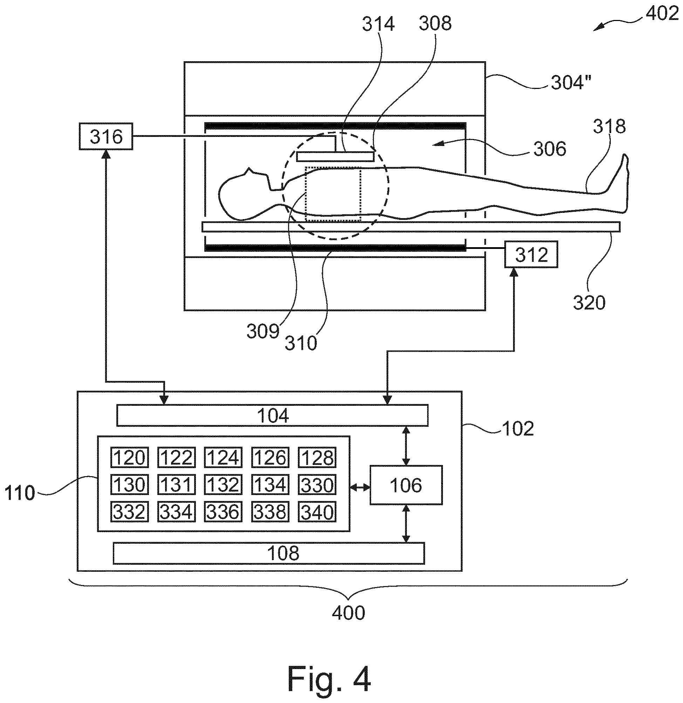

Fig. 4 illustrates a further example of a medical system 400. The medical system 400 in Fig. 4 is similar to the medical system 300 in Fig. 3 except instead of having two separate magnets 304, 304' there is only a single magnetic resonance imaging system 402. In this case the magnet 304" is a variable magnetic field magnet 304". Between the acquisitions the B0 magnetic field within the imaging zone 308 can be varied. This may have the advantage that the subject 318 is able to be imaged in the same location. Although there may be some registration which may be performed, the position of the subject 318 between the acquisition of the k-space data 336, 338, 340 will be much more consistent. The variable magnetic field magnet 304" may for example have a current which can be varied in between the acquisitions. The multi-b diffusion weighted k-space data 340 may be acquired either with the B0 field set to the first value or the second value.

The theoretical aspects of calculating an intra-cellular conductivity map 134 are now discussed in detail. Tissue conductivity is determined by the local concentration and mobility of ions. It can be measured with MR-based Electrical Properties Tomography (EPT). This conductivity relates (a) to the Larmor frequency (RF) of the MR system applied, and (b) to the total tissue conductivity comprising the intra- and extra-cellular tissue fraction.

Embodiments may separate the calculation of intra-cellular conductivity 134 and optionally an extra-cellular conductivity 138 by first deriving a low frequency conductivity map (126) (or LF conductivity) from measured RF conductivity (the first electrical properties tomography conductivity map 122 and second electrical properties tomography conductivity map 124) via a Cole-Cole-model as described previously. In a second step, a multi-b DWI scan is performed to estimate extra-volume fraction and optionally an intra-cellular volume fraction. In a third step, RF and LF conductivity as well as extra/intra-cellular volume fraction is used to estimate intra/extra-cellular fraction (intra-cellular volume fraction map 132 and extra-cellular volume fraction map 136) of the RF conductivity.

There are several different approaches to derive the low-frequency (LF) conductivity (corresponding to roughly 1-500 kHz) from the measured RF conductivity. In one approach, LF conductivity is derived from RF conductivity measured at 3T, together with multi-b diffusion weighted imaging (DWI), also measured at 3T. In another approach, LF conductivity is derived via a simplified Cole-Cole-model from two different RF conductivity maps, measured at 1.5 T and at 3T, but without taking DWI into account.

Examples described herein may have the benefit that the examples separate intra- and extra-cellular conductivity in vivo. Although intra- and extra-cellular ion mobility has been approximated from measurement-based estimation of intra- and extra-cellular diffusion constant, but intra- and extra-cellular ion concentration has only been taken from literature. Thus, no patient-individual, local separation of intra- and extra-cellular conductivity has been possible with this technique, or any other yet published approach.

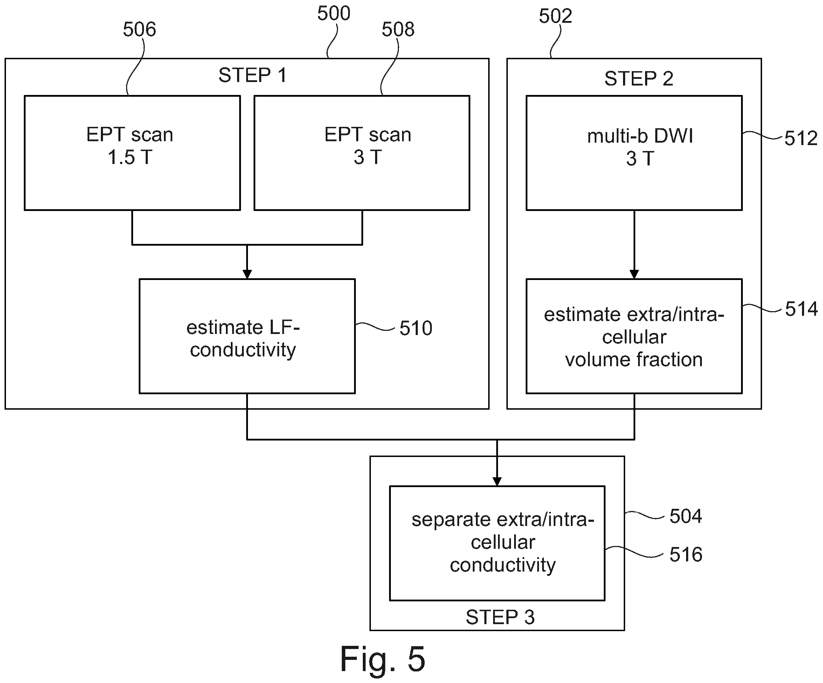

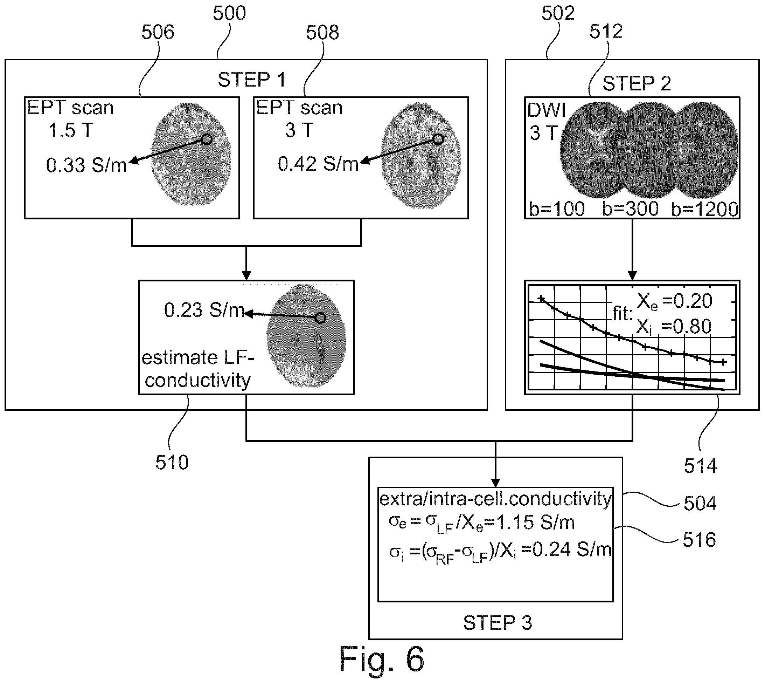

Examples described herein may separate intra- and extra-cellular conductivity by first deriving LF conductivity from measured RF conductivity via the simplified Cole-Cole-model. In a second step, a multi-b DWI scan is performed to estimate extra/intra-cellular volume fraction. In a third step, RF and LF conductivity as well as extra/intra-cellular volume fraction is used to estimate intra/extra-cellular fraction of the RF conductivity. These steps are described in detail in the following and sketched in Figs. 5 and 6 below.

Fig. 5 illustrates a schematic sketch of a method according to an example. The method is roughly divided into a first step 500, a second step 502 and a third step 504. In step 1 there are first two electrical properties tomography acquisitions. 506 represents for example performing an electrical properties tomography scan at 1.5T. This would correspond to acquiring the first electrical properties tomography k-space data 336. Step 508 represents for example performing an electrical properties tomography scan at 3T. This corresponds to acquiring the second electrical properties tomography k-space data 338. The information from these two scans is then used to estimate the low-frequency conductivity 510. This corresponds to step 204 of the method illustrated in Fig. 2.

Next, in step 2, a multi-b diffusion weighted image is performed at 3T 512. This corresponds to acquiring the multi-b diffusion weighted k-space data 340. In step 514 the extra- and intra-cellular volume fraction is estimated or calculated. This corresponds to steps 208 and 210 and step 214 of the method in Fig. 2. After steps 1 and 2 have been performed, the method proceeds to step 3 504. In step 516 the extra-cellular conductivity and the intra-cellular conductivity are calculated. This corresponds to step 212 and step 216 of Fig. 2.

Fig. 6 shows the same method as illustrated in Fig. 5 except this time the data of a subject is used for an illustration. For the area indicated in the interior white matter, an extra- and intra-cellular conductivity of 0.23 S/m and 1.28 S/m is calculated.

The steps illustrated in Figs. 5 and 6 is described in more detail below:

Step 1: Deriving LF conductivity from measured RF conductivity A 4-Cole-Cole-model of $\alpha/\beta/\gamma/\delta$-dispersion (as described in Gabriel S, Lau RW, Gabriel C. The dielectric properties of biological tissues: III. Parametric models for the dielectric spectrum of tissues. Phys Med Biol. 1996;41:2271-2293) is simplified such that a comparison of two RF conductivities measured at 64 MHz and 128 MHz yields the extrapolated LF conductivity $\sigma_{LF}$ at around 100 kHz. The two conductivity maps may be registered before extrapolation. No Diffusion Weighted Image (DWI) scan is required for this first step.

Step 2: Deriving intra/extra-cellular volume fraction In the second step, a multi-b DWI scan is performed and the resulting spectra S(b) are fitted voxel-by-voxel to two exponentials modelling the intra- and extra-cellular contribution

$$S(b)/S_0 = v_i \exp(-b\ d_i) + v_e \exp(-b\ d_e) + v_0 \qquad (1)$$

with $S_0$ a system dependent scaling factor, $d_i/d_e$ the intra/extra-cellular diffusion constant, and $v_i/v_e$ representing the intra/extra-cellular volume. $v_i$ is the intra-cellular volume map 130 and $v_e$ is the extra-cellular volume map 131. The measurement offset $v_0$ is a model refinement which optionally can be set to zero to facilitate data processing. Intra/extra-cellular volume fraction $X_i/X_e$ is given by:

$$X_i = v_i/(v_e+v_i)\ , \qquad (2a)$$

$$X_e = v_e/(v_e+v_i)\ . \qquad (2b)$$

$X_i$ is the intra-cellular volume fraction map 132 and $X_e$ is the extra-cellular volume fraction map 136.

Equation (1) is an example of a two-term exponential model. As used herein two-term exponential model referrers to an exponential equation with two exponential terms that are added together. The solution of Equation (1) using an optimization yields a solution to 4 unknowns, however some terms may be ignored after the optimization. The spatially dependent spectra S(b) above is the multi-b diffusion weighted magentic resonance imaging signal spectra map 128.

Step 3: Deriving intra/extra-cellular conductivity

RF conductivity $\sigma_{RF}$ can be modelled as weighted superposition of $\sigma_i$ and $\sigma_e$

$$\sigma_{RF} = X_e\,\sigma_e + X_i\,\sigma_i . \tag{3}$$

It can be assumed that the lipid membranes of the cells act as insulators at LF, thus prohibiting the intra-cellular space from contributing to the total conductivity, and yielding:

$$\sigma_{LF} = X_e\,\sigma_e . \tag{4}$$

Thus, the extra-cellular conductivity (extra-cellular conductivity map 138) is given by:

$$\sigma_e = \sigma_{LF}\,/\,X_e , \tag{5}$$

and correspondingly the intra-cellular conductivity (intra-cellular conductivity map 134):

$$\sigma_i = (\sigma_{RF} - \sigma_{LF})\,/\,X_i , \tag{6}$$

Additionally, intra/extra-cellular conductivity $\sigma_i\,/\,\sigma_e$ may be modelled as

$$\sigma_i = k\,d_i\,c_i \tag{7a}$$

$$\sigma_e = k\,d_e\,c_e \tag{7b}$$

with $c_i/c_e$ the intra/extra-cellular ion concentration and $k\,d_i/k\,d_e$ the intra/extra-cellular ion mobility, which is assumed to be proportional to the experimentally determined (water) diffusion constants, and some constant k. The described method allows to determine the ratio $\beta$ of intra/extra-cellular ion concentrations

$$\beta = c_i\,/\,c_e = (\sigma_{RF}\,/\,\sigma_{LF} - 1)\,(X_e\,d_e)\,/\,(X_i\,d_i). \tag{8}$$

It should be noted that previously $\beta$ has been taken from literature, i.e., the same value is used for all patients, all tissue types, and all pathological states. In contrast as described herein, $\beta$ is determined experimentally voxel-by-voxel, i.e., patient-individual and specific for the local tissue type and its pathologic state.

To obtain the absolute intra/extra-cellular ion concentrations given by Eq. (7), the constant k can be calculated via the tissue temperature, which can also be measured independently with MRI, as well as mean ion charge and the constants of Avogadro and Boltzmann ("Einstein-Smoluchowski relation").

It should further be noted that above mentioned quantities like ion concentration and ion mobility are based on a superposition of all ions contributing to conductivity, which is expected to be mainly sodium, but also other ions like potassium, calcium, chlorine, etc.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

LIST OF REFERENCE NUMERALS

| 100 | medical system |
| 102 | computer |
| 104 | hardware interface |
| 106 | computational system |
| 108 | user interface |
| 110 | memory |
| 120 | machine executable instrucctions |
| 122 | first electrical properties tomography conductivity map |
| 124 | second electrical properties tomography conductivity map |
| 126 | low frequency conductivity map |
| 128 | multi-b diffusion weighted magnetic resonance imaging signal spectra map |
| 130 | intra-cellular volume map |
| 131 | extra-cellular volume map |
| 132 | intra-cellular volume fraction map |
| 134 | intra-cellular conductivity map |
| 136 | extra-cellular volume fraction map |
| 138 | extra-cellular conductivity map |
| 200 | receive first electrical properties tomography conductivity map descriptive of a region of interest of a subject |
| 202 | receive a second electrical properties tomography conductivity map descriptive of the region of interest of a subject |
| 204 | calculate a low frequency conductivity map using the first electrical properties tomography conductivity map and the second electrical properties tomography conductivity map |
| 206 | receive a multi-b diffusion weighted magnetic resonance imaging signal spectra map |
| 208 | calculate an intra-cellular volume map and an extra-cellular volume map |
| 210 | calculate an intra-cellular volume fraction map by performing a voxel wise division of the intra-cellular volume map by the voxel wise sum of the extra-cellular volume map plus the intra-cellular volume map |
| 212 | calculate an intra-cellular conductivity map by performing a voxel wise division of an intermediate mapping by the intra-cellular volume fraction map |
| 214 | calculate an extra-cellular volume fraction map by performing a voxel wise division of the intra-cellular volume map by the voxel wise sum of the extra-cellular volume map plus the intra-cellular volume map |
| 216 | calculate an extra-cellular conductivity map by performing a voxel wise division of the low frequency conductivity map by the extra-cellular volume fraction map |
| 300 | medical system |
| 302 | first magnetic resonance imaging system |
| 302' | second magnetic resonance imaging system |
| 302" | variable B0 field magentic resonance imaging system |
| 304 | first magnet |
| 304' | second magnet |
| 304" | variable magnetic field magnet |
| 306 | bore of magnet |
| 308 | imaging zone |
| 309 | region of interest |
| 310 | magnetic field gradient coils |
| 312 | magnetic field gradient coil power supply |
| 314 | radio-frequency coil |
| 316 | transceiver |
| 318 | subject |
| 320 | subject support |
| 330 | first pulse sequence commands |
| 332 | second pulse sequence commands |

| | |
|---|---|
| 334 | third pulse sequence commands |
| 336 | first electrical properties tomography k-space data |
| 338 | second electrical properites tomography k-space data |
| 340 | multi-b diffusion weighted k-space data |
| 400 | medical system |
| 500 | step 1 |
| 502 | step 2 |
| 504 | step 3 |
| 506 | first EPT scan |
| 508 | second EPT scan |
| 510 | estimate low frequency conductivity map |
| 512 | perform DWI MRI |
| 514 | estimate extra/intra-cellular volume fraction |
| 516 | separate extra/intra-cellular conductivity |

**Claims**

1. A medical system (100, 300, 400) comprising:

   - a memory (110) storing machine executable instructions (120);
   - a computational system (106) configured for controlling the medical system, wherein execution of the machine executable instructions causes the computational system to:

      - receive (200) a first electrical properties tomography conductivity map (122) descriptive of a region of interest (309) of a subject (318), wherein the first electrical properties tomography conductivity map is for a first radio frequency value;
      - receive (202) a second electrical properties tomography conductivity map (124) descriptive of the region of interest of the subject, wherein the second electrical properties tomography conductivity map is for a second radio frequency value, wherein the first electrical properties tomography conductivity map is registered to the second electrical properties tomography conductivity map;
      - calculate (204) a low frequency conductivity map (126) using the first electrical properties tomography conductivity map and the second electrical properties tomography conductivity map;
      - receive (206) a multi-b diffusion weighted magnetic resonance imaging signal spectra map (128), wherein the multi-b diffusion weighted magnetic resonance imaging signal spectra map is registered to the first electrical properties tomography conductivity map or the second electrical properties tomography conductivity map;
      - calculate (208) an intra-cellular volume map (130) and an extra-cellular volume map (131), by performing an optimization of a two-term exponential model to the multi-b diffusion weighted magnetic resonance imaging signal spectra map;
      - calculate (210) an intra-cellular volume fraction map (132) by performing a voxel wise division of the intra-cellular volume map by the voxel wise sum of the extra-cellular volume map plus the intra-cellular volume map; and
      - calculate (212) an intra-cellular conductivity map (134) by performing a voxel wise division of an intermediate mapping by the intra-cellular volume fraction map, wherein the intermediate mapping is the voxel wise difference of a selected radio frequency conductivity map and the low frequency conductivity map, wherein the selected radio frequency conductivity map is one of the first electrical properties tomography conductivity map and the second electrical properties tomography conductivity map.

2. The medical system of claim 1, wherein execution of the machine executable instructions further causes the computational system to:

   - calculate (214) an extra-cellular volume fraction map (136) by performing a voxel wise division of the intra-cellular volume map by the voxel wise sum of the extra-cellular volume map plus the intra-cellular volume map; and
   - calculate (216) an extra-cellular conductivity map (138) by performing a voxel wise division of the low frequency conductivity map by the extra-cellular volume fraction map.

3. The medical system of claim 1 or 2, wherein execution of the machine executable instructions further causes the computational system to render the intra-cellular conductivity map.

4. The medical system of claim 3, wherein the intra-cellular conductivity map is rendered as an overlay over a magnetic resonance image, and wherein the intra-cellular conductivity map is registered to the magnetic resonance image.

5. The medical system of any one of the preceding claims, wherein the calculation of the low frequency conductivity map using the first electrical properties tomography conductivity map and the second electrical properties tomography conductivity map is performed as an extrapolation according to a Cole-Cole model.

6. The medical system of any one of the preceding claims, wherein the optimization of the two-term exponential model to the multi-b diffusion weighted magnetic resonance imaging signal spectra map also provides an intra-cellular diffusion constant map and an extra-cellular diffusion constant map,
wherein execution of the machine executable instructions further causes the computational system to perform any one of the following:

- calculate an intra-cellular ion concentration map using the intra-cellular conductivity map and the intra-cellular diffusion constant map;
- calculate an extra-cellular ion concentration map using the extra-cellular conductivity map and the extra-cellular diffusion constant map; and
- combinations thereof.

7. The medical system of claim 6, wherein the intra-cellular ion concentration map and/or the inter-cellular ion concentration map are temperature corrected using any one of the following: a constant body temperature of the subject and a magnetic resonance thermometry temperature map of the region of interest.

8. The medical system of any one of the preceding claims, wherein execution of the machine executable instructions further causes the computational system to:

- receive first electrical properties tomography k-space data (336) descriptive of the region of interest for a first proton resonance frequency matching the first radio frequency value;
- receive second electrical properties tomography k-space data (338) descriptive of the region of interest for a second proton resonance frequency matching the second radio frequency value;
- receive multi-b diffusion weighted k-space data (340) descriptive of the region of interest with either the first proton resonance frequency or the second proton resonance frequency;
- reconstruct the first electrical properties tomography conductivity map from the first electrical properties tomography k-space data;
- reconstruct the second electrical properties tomography conductivity map from the second electrical properties tomography k-space data; and
- reconstruct the multi-b diffusion weighted magnetic resonance imaging signal spectra map from the multi-b diffusion weighted k-space data.

9. The medical system of claim 7, wherein execution of the machine executable instructions further causes the computational system to register the first electrical properties tomography conductivity map, the second electrical properties tomography conductivity map, and the multi-b diffusion weighted magnetic resonance imaging signal spectra map to each other.

10. The medical system of claim 8, wherein the medical system further comprises a magnetic resonance imaging system (302, 202', 302") configured for acquiring k-space data for at least two distinct B0 magnetic field values, wherein the at least two distinct magnetic field values have a proton resonance frequency matching the first radio frequency value and the second radio frequency value, wherein the memory further contains first pulse sequence commands (330) configured for controlling the magnetic resonance imaging system to acquire the first electrical properties tomography k-space data, wherein the memory further contains second pulse sequence commands (332) configured for controlling the magnetic resonance imaging system to acquire the second electrical properties tomography k-space data, wherein the memory further contains third pulse sequence commands (334) configured for controlling the magnetic resonance imaging system to acquire the multi-b diffusion weighted k-space data, wherein execution of the machine executable instructions further causes the computational system to control the magnetic resonance imaging system to:

- acquire the first electrical properties tomography k-space data by controlling the magnetic resonance imaging system with the first pulse sequence commands;
- acquire the second electrical properties tomography k-space data by controlling the magnetic resonance imaging system with the second pulse sequence commands; and
- acquire the multi-b diffusion weighted k-space data by controlling the magnetic resonance imaging system with the third pulse sequence commands.

11. The medical system of claim 10, wherein the magnetic resonance imaging system comprises a main magnet (302, 302') for each of the at least two distinct B0 magnetic field values.

12. The medical system of claim 10, wherein the magnetic resonance imaging system comprises a variable magnetic field main magnet (302") configured for adjusting the B0 magnetic field to each of the at least two distinct B0 magnetic field values.

13. A method of medical imaging, wherein the method comprises:

- receiving (200) a first electrical properties tomography conductivity map (122) descriptive of a region of interest (309) of a subject (318), wherein the first electrical properties tomography conductivity map is for a first radio frequency value;
- receiving (202) a second electrical properties tomography conductivity map (124) descriptive of the region of interest of the subject wherein the second electrical properties tomography conductivity map is for a second radio frequency value, wherein the first electrical properties tomography conductivity map is registered to the second electrical properties tomography conductivity map;
- calculating (204) a low frequency conductivity map (126) using the first electrical properties tomography conductivity map and the second electrical properties tomography conductivity map;
- receiving (206) a multi-b diffusion weighted magnetic resonance imaging signal spectra map (128), wherein the multi-b diffusion weighted magnetic resonance imaging signal spectra map is registered to the first electrical properties tomography conductivity map or the second electrical properties tomography conductivity map
- calculating (208) an intra-cellular volume map (130) and an extra-cellular volume map (131), by performing an optimization of a two-term exponential model to the multi-b diffusion weighted magnetic resonance imaging signal spectra map;
- calculating (210) an intra-cellular volume fraction map (132) by performing a voxel wise division of the intra-cellular volume map by the voxel wise sum of the extra-cellular volume map plus the intra-cellular volume map; and
- calculating (212) an intra-cellular conductivity map (134) by performing a voxel wise division of an intermediate mapping by the intra-cellular volume fraction map, wherein the intermediate mapping is the voxel wise difference of a selected radio frequency conductivity map and the low frequency conductivity map, wherein the selected radio frequency conductivity map is one of the first electrical properties tomography conductivity map and the second electrical properties tomography conductivity map.

14. A computer program comprising machine executable instructions (120) for execution by a computational system (106) controlling a medical system (100, 300, 400), wherein execution of the machine executable instructions causes the computational system to:

- receive (200) a first electrical properties tomography conductivity map (122) descriptive of a region of interest (309) of a subject (318), wherein the first electrical properties tomography conductivity map is for a first radio frequency value;
- receive (202) a second electrical properties tomography conductivity map (124) descriptive of the region of interest of the subject wherein the second electrical properties tomography conductivity map is for a second radio frequency value, wherein the first electrical properties tomography conductivity map is registered to the second electrical properties tomography conductivity map;
- calculate (204) a low frequency conductivity map (using the first electrical properties tomography conductivity map and the second electrical properties tomography conductivity map;
- receive (206) a multi-b diffusion weighted magnetic resonance imaging signal spectra map (128), wherein the multi-b diffusion weighted magnetic resonance imaging signal spectra map is registered to the first electrical properties tomography conductivity map or the second electrical properties tomography conductivity map
- calculate (208) an intra-cellular volume map (130) and an extra-cellular volume map (131), by performing an optimization of a two-term exponential model to the multi-b diffusion weighted magnetic resonance imaging signal spectra map;

- calculate (210) an intra-cellular volume fraction map (130) by performing a voxel wise division of the intra-cellular volume map by the voxel wise sum of the extra-cellular volume map plus the intra-cellular volume map; and

- calculate (212) an intra-cellular conductivity map (132) by performing a voxel wise division of an intermediate mapping by the intra-cellular volume fraction map, wherein the intermediate mapping is the voxel wise difference of a selected radio frequency conductivity map and the low frequency conductivity map, wherein the selected radio frequency conductivity map is one of the first electrical properties tomography conductivity map and the second electrical properties tomography conductivity map.

Fig. 1

```
┌─────────────────────────────────────────┐
│  receive a first electrical properties   │
│      tomography conductivity map         │──── 200
└─────────────────────────────────────────┘
                    │
                    ▼
┌─────────────────────────────────────────┐
│  receive a second electrical properties  │
│      tomography conductivity map         │──── 202
└─────────────────────────────────────────┘
                    │
                    ▼
┌─────────────────────────────────────────┐
│   calculate a low frequency conductivity │
│                  map                     │──── 204
└─────────────────────────────────────────┘
                    │
                    ▼
┌─────────────────────────────────────────┐
│  receive a multi-b diffusion weighted    │
│  magnetic resonance imaging signal       │
│            spectra map                   │──── 206
└─────────────────────────────────────────┘
                    │
                    ▼
┌─────────────────────────────────────────┐
│   calculate an intra-cellular volume map │
│      and an extra-cellular volume map    │──── 208
└─────────────────────────────────────────┘
                    │
                    ▼
┌─────────────────────────────────────────┐
│    calculate an intra-cellular volume    │
│              fraction map                │──── 210
└─────────────────────────────────────────┘
                    │
                    ▼
┌─────────────────────────────────────────┐
│  calculate an intra-cellular conductivity│
│                  map                     │──── 212
└─────────────────────────────────────────┘
                    │
                    ▼
┌─────────────────────────────────────────┐
│    calculate an extra-cellular volume    │
│              fraction map                │──── 214
└─────────────────────────────────────────┘
                    │
                    ▼
┌─────────────────────────────────────────┐
│  calculate an extra-cellular conductivity│
│                  map                     │──── 216
└─────────────────────────────────────────┘
```

Fig. 2

Fig. 3

Fig. 4

Fig. 5

STEP 1

506 — EPT scan
1.5 T

0.33 S/m

508 — EPT scan
3 T

0.42 S/m

0.23 S/m

estimate LF-conductivity

510

STEP 2

512 — DWI
3 T

b=100    b=300    b=1200

fit: $X_e$=0.20
$X_i$=0.80

514

extra/intra-cell.conductivity
$\sigma_e = \sigma_{LF}/X_e = 1.15$ S/m
$\sigma_i = (\sigma_{RF} - \sigma_{LF})/X_i = 0.24$ S/m

504

516

STEP 3

Fig. 6

EP 3 892 191 A1

Europäisches Patentamt

European Patent Office

Office européen des brevets

# EUROPEAN SEARCH REPORT

Application Number

EP 20 16 8971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | SAJIB SAURAV Z ET AL: "Electrodeless conductivity tensor imaging (CTI) using MRI: basic theory and animal experiments", BIOMEDICAL ENGINEERING LETTERS, THE KOREAN SOCIETY OF MEDICAL AND BIOLOGICAL ENGINEERING, KOREA, vol. 8, no. 3, 25 April 2018 (2018-04-25), pages 273-282, XP036556369, ISSN: 2093-9868, DOI: 10.1007/S13534-018-0066-3 [retrieved on 2018-04-25] * the whole document * | 1-14 | INV. A61B5/053 G01R33/56 G01R33/563 ADD. G01R33/44 |
| Y | ULRICH KATSCHER, CHRISTOPH LEUSSLER, NICK FLAESCHNER, FABIAN WENZEL: "Estimation of low frequency conductivity from Larmor frequency conductivity", INTERNATIONAL SOCIETY FOR MAGNETIC RESONANCE IN MEDICINE, ISMRM, 2030 ADDISON STREET, 7TH FLOOR, BERKELEY, CA 94704 USA, 0695, 26 April 2019 (2019-04-26), XP040708082, * the whole document * | 1-14 | |
| A | JAKOB GEORGI ET AL: "Influence of the extracellular matrix on water mobility in subcortical gray matter", MAGNETIC RESONANCE IN MEDICINE., vol. 81, no. 2, 14 September 2018 (2018-09-14), pages 1265-1279, XP055733082, US ISSN: 0740-3194, DOI: 10.1002/mrm.27459 * the whole document * | 1-14 | TECHNICAL FIELDS SEARCHED (IPC) A61B G01R |

-/--

The present search report has been drawn up for all claims

| Place of search | Date of completion of the search | Examiner |
|---|---|---|
| Munich | 25 September 2020 | Vanhaecke, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 16 8971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | QIANG ZENG ET AL: "A Modified Tri-Exponential Model for Multi-b-value Diffusion-Weighted Imaging: A Method to Detect the Strictly Diffusion-Limited Compartment in Brain", FRONTIERS IN NEUROSCIENCE, vol. 12, 26 February 2018 (2018-02-26), XP055733123, CH ISSN: 1662-4548, DOI: 10.3389/fnins.2018.00102 * the whole document * | 1-14 | |
| Y | MUN BAE LEE ET AL: "Extracellular electrical conductivity property imaging by decomposition of high-frequency conductivity at Larmor-frequency using multi-b-value diffusion-weighted imaging", PLOS ONE, vol. 15, no. 4, 8 April 2020 (2020-04-08), page e0230903, XP055690471, DOI: 10.1371/journal.pone.0230903 * the whole document * | 1-14 | TECHNICAL FIELDS SEARCHED (IPC) |
| A | KATSCHER U ET AL: "Determination of Electric Conductivity and Local SAR Via B1 Mapping", IEEE TRANSACTIONS ON MEDICAL IMAGING, IEEE SERVICE CENTER, PISCATAWAY, NJ, US, vol. 28, no. 9, 1 September 2009 (2009-09-01), pages 1365-1374, XP011268263, ISSN: 0278-0062, DOI: 10.1109/TMI.2009.2015757 * the whole document * | 8,10 | |

The present search report has been drawn up for all claims

| Place of search | Date of completion of the search | Examiner |
|---|---|---|
| Munich | 25 September 2020 | Vanhaecke, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAJIB.** Electrodeless conductivity tensor imaging (CTI) using MRI: basic theory and animal experiments. *Biomedical Engineering Letters,* 25 April 2018, vol. 8 (3), 273-282

- **GABRIEL S ; LAU RW ; GABRIEL C.** The dielectric properties of biological tissues: III. Parametric models for the dielectric spectrum of tissues. *Phys Med Biol.,* 1996, vol. 41, 2271-2293